# FASCICULE DE BREVET EUROPEEN

(11) **EP 1 259 283 B1**
(45) Date de publication et mention de la délivrance du brevet: **10.08.2005**
(21) Numéro de dépôt: 01907824.5
(22) Date de dépôt: 16.02.2001
(51) Int. Cl.: A61M 39/02

(54) **DISPOSITIF IMPLANTABLE D'INJECTION DE SUBSTANCES MEDICALES**
IMPLANTIERBARE VORRICHTUNG ZUR ARZNEIMITTELINJEKTION
IMPLANTABLE DEVICE FOR INJECTING MEDICAL SUBSTANCES

(30) Priorité: 18.02.2000 FR 0002074
(43) Date de publication de la demande: 27.11.2002
(73) Titulaire: Compagnie Européenne d'Etude et de Recherche de Dispositifs pour l'Implantation par Laparoscopie, 38200 Vienne (FR)
(72) Inventeur: BENCHETRIT, Salomon, F-69300 Caluire (FR)
(74) Mandataire: Martin, Didier Roland Valéry
(86) Numéro de dépôt international: PCT/FR2001/000465
(87) Numéro de publication internationale: WO 2001/060444

(56) Documents cités:
- WO-A-99/16501
- US-A- 4 781 680
- US-A- 4 840 615
- US-A- 5 137 529
- US-A- 5 460 612

## Description

La présente invention se rapporte au domaine technique des dispositifs d'injection de substances médicales destinés à être implantés dans une zone sous-cutanée du corps d'un patient, pour réaliser des injections desdites substances dans le corps du patient. Un dispositif selon le préambule de la revendication 1 est connu du document US-A-4 840 615.

De tels dispositifs d'injection sont encore appelés sites implantables.

La présente invention concerne un dispositif d'injection du type comprenant un boîtier rigide creux, qui comporte un fond à partir duquel s'étendent des parois latérales dont les extrémités libres définissent une ouverture proximale, une membrane qui est située au niveau des extrémités libres des parois latérales pour fermer de manière sensiblement hermétique l'ouverture proximale, délimitant ainsi une chambre, et un conduit connecté au boîtier rigide et qui relie la chambre à l'extérieur du dispositif d'injection.

### TECHNIQUE ANTERIEURE

Lors d'une injection, la membrane, qui est accessible au travers de la peau du patient, est destinée à être transpercée par une aiguille pour envoyer une dose de la substance médicale dans le corps du patient au travers de la chambre et du conduit. Afin d'éviter la pénétration de l'aiguille dans le corps du patient lors de son introduction dans la chambre, les parois latérales et le fond du boîtier sont non transperçables.

Généralement, le conduit est relié à un cathéter qui permet de délivrer directement la substance médicale dans la région du corps qui doit être traitée. Ces dispositifs d'injection peuvent demeurer dans le corps du patient pendant des périodes prolongées afin d'éviter, lors de traitement médicaux lourds, des injections intra-veineuses, intra-artérielles ou bien encore intra-rachidiennes répétées, en les remplaçant par de simples injections sous-cutanées. Les traumatismes physiologiques et psychologiques du patient sont ainsi réduits.

Toutefois, les normes médicales imposent que le boîtier soit suffisamment rigide pour qu'il n'y ait aucun risque, lors d'une injection, que l'aiguille n'atteigne le corps du patient lorsqu'elle est enfoncée au travers de la membrane. Par conséquent, les dispositifs d'injection actuellement connus créent encore des traumatismes du corps du patient en raison de leur rigidité.

Par ailleurs, dans les sites implantables couramment rencontrés, la membrane est généralement immobilisée par tout moyen de blocage mécanique connu au niveau des extrémités libres des parois latérales du boîtier rigide. Même si le blocage de la membrane est initialement réalisé afin de rendre la chambre du dispositif d'injection hermétique, l'utilisation prolongée de ce dispositif entraîne une détérioration de ce blocage et donc des risques de fuite et de désinsertion totale de la membrane. Le dispositif doit alors être changé pour être remplacé par un nouveau dispositif, ce qui oblige à réaliser une nouvelle intervention chirurgicale, et crée donc un désagrément notable pour le patient.

### EXPOSE DE L'INVENTION

L'objet assigné à l'invention vise en conséquence à proposer un nouveau dispositif d'injection de substances médicales permettant de remédier aux différents inconvénients énumérés précédemment, en fournissant un dispositif qui soit atraumatique tout en supprimant les risques de fuite et de déblocage de la membrane, de conception simple et qui facilite l'opération d'injection en général.

Un autre objet de l'invention vise à proposer un nouveau dispositif d'injection qui facilite l'introduction de l'aiguille dans la chambre du boîtier, afin d'éviter que l'utilisateur de cette aiguille doive faire plusieurs essais au travers de la peau du patient pour localiser la membrane. La palpation de la peau du patient ne permet pas en effet de toujours localiser de façon certaine la surface de la membrane. Aussi, le dispositif proposé permet d'atteindre la chambre en toute sécurité, même si l'aiguille est introduite de manière inclinée au travers de l'épaisseur de cette membrane.

Encore un autre objet de l'invention vise à proposer un nouveau dispositif d'injection particulièrement stable avec un nombre réduit d'éléments.

Un autre objet de l'invention vise à proposer un nouveau dispositif d'injection qui peut être facilement immobilisé dans le corps du patient.

Les objets assignés à l'invention sont atteints à l'aide d'un nouveau dispositif d'injection de substances médicales destiné à être implanté dans une zone sous-cutanée du corps du patient pour réaliser des injections desdites substances dans le corps, le dispositif comprenant :
- un boîtier rigide creux qui comporte un fond à partir duquel s'étendent des parois latérales dont les extrémités libres définissent une ouverture proximale,
- une membrane qui est située au niveau des extrémités libres des parois latérales pour fermer de manière sensiblement hermétique l'ouverture proximale, délimitant ainsi une chambre,
- un conduit connecté au boîtier rigide et qui relie la chambre à l'extérieur du dispositif d'injection,
la membrane étant, lors d'une injection, destinée à être transpercée par une aiguille pour envoyer une dose de ladite substance dans le corps du patient au travers de la chambre et dudit conduit,
les parois latérales et le fond du boîtier rigide étant non transperçables par l'aiguille pour empêcher la pénétration de l'aiguille dans le corps du patient lors de son introduction dans la chambre,
le dispositif comprenant en outre une enveloppe en matériau élastomère qui recouvre le boîtier, la membrane faisant partie intégrante de l'enveloppe, et le grade de l'élastomère utilisé pour la membrane étant différent de celui de l'enveloppe.

### DESCRIPTIF SOMMAIRE DES DESSINS

D'autres objets et avantages de l'invention apparaîtront mieux à la lecture de la description ci-jointe, ainsi qu'à l'aide des dessins annexés, à titre purement illustratif et informatif, dans lesquels :
- La figure 1 est une vue schématique en perspective d'un premier mode de réalisation d'un dispositif d'injection conforme à l'invention.
- Les figures 2 et 3 illustrent respectivement une vue de dessus et une vue en coupe transversale du dispositif d'injection de la figure 1.
- La figure 4 est une vue en coupe transversale du boîtier et du conduit du dispositif de la figure 1.
- La figure 5 est une vue en coupe transversale d'un deuxième mode de réalisation d'un dispositif d'injection conforme à l'invention.
- Les figures 6 et 7 illustrent respectivement, selon des vues de dessus et en coupe transversale, un troisième mode de réalisation d'un dispositif d'injection conforme à la présente invention.

Sur les différentes figures, les éléments identiques ou similaires sont désignés par les mêmes références.

### MEILLEURE MANIERE DE REALISER L'INVENTION

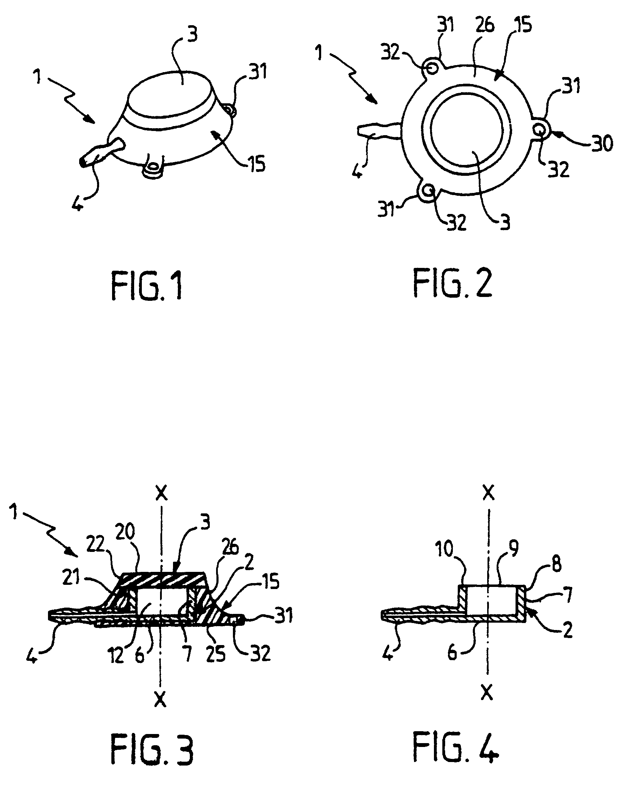

Les figures 1 à 4 illustrent un exemple de réalisation préférentielle d'un dispositif d'injection 1 de substances médicales conformes à l'invention et destiné à être implanté dans une zone sous-cutanée du corps d'un patient pour réaliser des injections de ces substances dans le corps.

De manière connue en soi, le dispositif 1 comprend un corps rigide creux 2 qui est fermé par une membrane 3 et auquel est connecté un conduit 4. La membrane 3 est couramment appelée « septum ».

Le corps rigide creux 2 est réalisé d'un seul tenant et comporte un fond 6 à partir duquel s'étendent des parois latérales 7 dont les extrémités supérieures 8 sont libres et délimitent une ouverture supérieure 9. Cette ouverture est également appelée ouverture proximale dans la mesure où, lorsque le dispositif est implanté sous la peau du patient, cette ouverture débouche juste au-dessous de la peau.

De manière préférentielle, le fond 6 du boîtier 2 est en forme de disque, de sorte que le boîtier se présente sous la forme d'un cylindre d'axe X-X et de section transversale circulaire, qui est fermé à l'une de ses extrémités et qui est débouchant à l'autre de ses extrémités. Il est réalisé en un matériau rigide tel que du titane.

Les extrémités supérieures libres 8 des parois latérales 7 sont d'une certaine épaisseur, définissant ainsi un anneau 10 qui s'étend parallèlement au fond 6 du boîtier 2.

La hauteur des parois latérales 7 du boîtier est faible par rapport au diamètre du fond 6 et est par exemple égale à la moitié.

Le conduit 4 est de faible diamètre et est rapporté sur le boîtier 2. De façon préférentielle, ce conduit est connecté aux parois latérales 7 du boîtier, au voisinage du fond 6. Il permet de relier le volume intérieur du boîtier 2 à l'extérieur du dispositif d'injection 1. Le conduit 4 est rigide et est également réalisé en titane par exemple.

Par ailleurs, la membrane 3 du dispositif d'injection 1 est immobilisée au niveau des extrémités libres 8 des parois latérales 7 du boîtier 2 pour fermer l'ouverture proximale 9 de manière sensiblement hermétique. Le fond 6 et les parois latérales 7 du boîtier 2 délimitent ainsi avec la membrane 3 une chambre 12 hermétiquement fermée et étanche.

De manière classique, la membrane 3 est réalisée en un matériau élastomère du type silicone ayant des propriétés *« auto-cicatrisantes ».* Ces propriétés font que, après avoir percé la membrane 3, l'orifice correspondant au perçage se rebouche automatiquement, ce qui ne détériore pas l'étanchéité de la chambre 12.

Cette membrane 3 est en effet, lors d'une injection d'une dose de substance médicale dans le corps du patient, destinée à être transpercée par une aiguille pour envoyer cette substance dans le corps du patient au travers de la chambre 12 et du conduit 4. Pour cela, le conduit 4 est relié à un cathéter (non représenté), lui-même monté débouchant dans la zone du corps où la substance doit être injectée.

Lorsque l'utilisateur de l'aiguille, par exemple l'infirmière, enfonce l'aiguille dans la membrane 3, le fond 6 et les parois latérales 7 du boîtier 2 sont suffisamment résistants pour interdire à l'aiguille de ressortir de la chambre 12 et pour éviter ainsi que l'extrémité de l'aiguille n'atteigne le corps du patient et ne crée une blessure interne dans le corps du patient.

Selon une caractéristique importante de l'invention, le dispositif d'injection 1 comprend une enveloppe 15 qui recouvre directement le boîtier 2. De manière avantageuse, l'enveloppe 15 s'étend sur toutes les faces externes du boîtier 2, c'est à dire sur la face inférieure du fond 6 opposée à l'ouverture proximale 9, ou au moins sur une fraction du fond 6 correspondant à un anneau périphérique sur ou autour du fond 6 et sur les faces externes des parois latérales 7 de ce boîtier. Seul le conduit 4 fait saillie hors de l'enveloppe 15 pour relier la chambre 12 à l'extérieur du dispositif d'injection 1.

L'enveloppe 15 est par exemple réalisée par moulage, par injection autour du boîtier rigide 2 d'un matériau élastomère du type silicone.

Selon la présente invention, la membrane 3 fait partie intégrante de l'enveloppe 15. Au sens de l'invention, on entend par partie intégrante le fait que la membrane 3 est intimement liée avec l'enveloppe 2, dans la mesure où elles sont réalisées ensemble autour du boîtier 2 lors du procédé d'injection. La membrane 3 et l'enveloppe 15 forment ainsi une pièce monobloc assimilable à un surmoulage sur et autour du boîtier 2. De préférence, la membrane 3 et l'enveloppe 15 sont réalisées dans le même type de matériau, c'est à dire en élastomère.

Seul le grade de l'élastomère utilisé pour l'enveloppe 15 est différent du grade de l'élastomère de la membrane 3. Lorsque, de manière préférentielle, le grade de la membrane 3 est différent du grade de l'enveloppe 15, il sera de préférence inférieur à celui de l'enveloppe 15 pour obtenir une membrane 3 de rigidité inférieure à la rigidité de l'enveloppe 15. Le dispositif d'injection 1 ainsi obtenu est de dureté variable. Il permet donc de minimiser le traumatisme subi par le corps du patient tout en facilitant la pénétration de l'aiguille dans la membrane 3, ainsi que le repérage tactile de la membrane 3 qui présente une rigidité moindre et différenciée par rapport au reste du dispositif d'injection 1.

On comprend donc que la membrane 3 n'est pas rapportée sur l'enveloppe 15 et n'est pas retenue sur celle-ci par des moyens positifs de blocage eux-mêmes rapportés. Elle est au contraire intimement liée à l'enveloppe 15 sans plan de séparation pour ne former qu'une seule masse de matériau indistincte. La membrane 3 ne peut donc se désolidariser de l'enveloppe 2, et il n'y a ainsi aucun risque de fuite, ni d'expulsion de la membrane 3.

Selon une autre caractéristique importante de l'invention, la membrane 3 repose et s'appuie directement sur les extrémités libres 8 et est conformée pour constituer une surépaisseur s'étendant au-dessus des extrémités libres 8 des parois latérales 7 du boîtier 2, en direction opposée au fond 6 de ce boîtier, pour être transperçable latéralement.

Dans une variante préférentielle, la membrane 3 s'étend entièrement au-dessus du boîtier 2, à partir des extrémités libres 8 des parois latérales 7. La membrane 3 prend ainsi la forme d'un cylindre de même axe X-X que le boîtier 2, et qui possède une face supérieure 20 et une face inférieure 21 entre lesquelles s'étendent des parois latérales 22. La hauteur des parois latérales 22 est sensiblement égale au tiers environ de la hauteur de l'ensemble du dispositif d'injection 1, et est par exemple de l'ordre de quelques millimètres.

La face inférieure 21 est en appui sur l'anneau 10 défini par les extrémités supérieures 8 des parois latérales 7 du boîtier 2. Les parois latérales 22 de la membrane 3 s'étendent dans le prolongement des parois latérales externes de l'enveloppe 15.

Ainsi, lors d'une injection, l'aiguille peut être introduite dans la chambre 12 non seulement par la face supérieure 20 de la membrane 3, mais aussi par les faces latérales 22 de cette membrane, puisque celle-ci possède une épaisseur qui est directement accessible sous la peau du patient. La probabilité d'introduire l'aiguille au bon endroit pour atteindre la chambre 12 est donc augmentée puisque l'aiguille peut être introduite dans cette chambre selon une grande variété d'angles par rapport à l'axe X-X du boîtier 2. Le travail de l'infirmière est donc rendu plus facile, en minimisant les risques de piquer le patient sans atteindre l'intérieur de la chambre 12.

De manière avantageuse encore, l'enveloppe 15 est d'épaisseur variable autour du boîtier 2 pour rendre le dispositif d'injection 1 atraumatique.

L'enveloppe 15 possède une embase 25 située au voisinage du fond 6 du boîtier 2, qui est de diamètre élargi par rapport à ce fond 6, et à partir de laquelle s'étendent des parois latérales externes 26 de forme convergente en direction de la membrane 3. Les parois latérales 26 sont par exemple de forme tronconique. La stabilité du dispositif 1 à l'intérieur du corps du patient est ainsi augmentée, ce qui minimise les risques de retournement de ce dispositif.

Afin d'ancrer fermement le dispositif 1 à l'intérieur du corps du patient, l'embase 25 de l'enveloppe 15 possède des moyens d'immobilisation 30 qui prennent, dans une première forme de réalisation, la forme de trois pattes 31 qui sont saillies à partir du pourtour de l'embase 25. A l'intérieur de ces pattes 31 sont ménagés des orifices 32. Les trois pattes 21 sont régulièrement réparties sur le pourtour de l'embase 25, et sont donc mutuellement écartées de 120°.

En variante, le dispositif 1 est muni d'une seule patte 31.

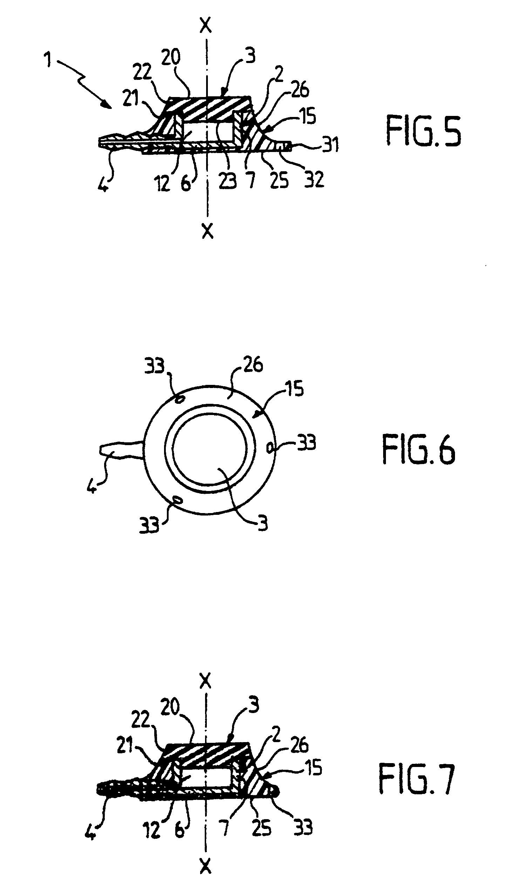

Le deuxième mode de réalisation représenté à la figure 5 diffère du premier mode de réalisation précédemment décrit uniquement par l'épaisseur de la membrane 3 qui est à présent variable.

L'épaisseur de la membrane 3, à l'aplomb de l'ouverture 9 du boîtier 2, est supérieure à l'épaisseur du reste de la membrane de manière à ancrer transversalement de manière ferme cette membrane dans le boîtier 2. La membrane 3 possède ainsi un corps central 23 qui pénètre légèrement dans l'ouverture du boîtier 2, formant un épaulement avec le reste de la membrane. Ceci évite un déplacement latéral trop important de la membrane 3 lorsque l'infirmière palpe le corps du patient pour localiser le dispositif 1 sous la peau de ce patient pour insérer l'aiguille.

Dans le troisième mode de réalisation représenté aux figures 6 et 7, les moyens d'immobilisation 30 du dispositif 1 prennent la forme de trois orifices 33 qui sont directement ménagés dans l'épaisseur du pourtour de l'embase 25.

Dans ce troisième mode de réalisation, le pourtour de l'embase 25 est, en section transversale, de forme arrondie pour être atraumatique.

De manière avantageuse, le diamètre de l'embase 25 n'est moins élargi que dans les premier et deuxième modes de réalisation pour minimiser les risques, lors du retrait du dispositif 1 hors du patient, de laisser des résidus de silicone à l'intérieur du corps.

Dans encore une autre variante, un biseau intérieur (non représenté) est ménagé sur l'anneau 10 défini par les extrémités libres 8 des parois latérales 7 du boîtier 2 pour favoriser la pénétration de l'aiguille dans la chambre 12 lorsque cette aiguille est enfoncée latéralement, par les parois latérales 22 de la membrane 3.

Le dispositif selon la présente invention possède donc une enveloppe 15 qui forme une seule pièce avec la membrane 3 et est d'une souplesse variable qui est adaptée au mieux pour que le dispositif d'injection 1 soit atraumatique pour le corps du patient.

Ce dispositif est par exemple particulièrement bien adapté pour les traitements chimiothérapeutiques.

### POSSIBILITE D'APPLICATION INDUSTRIELLE

L'invention trouve son application industrielle dans la réalisation et l'utilisation de dispositifs de site d'injection de substances médicales.

## Revendications

1. Dispositif d'injection de substances médicales destiné à être implanté dans une zone sous-cutanée du corps d'un patient pour réaliser des injections desdites substances dans le corps, le dispositif (1) comprenant :
- un boîtier rigide creux (2) qui comporte un fond (6) à partir duquel s'étendent des parois latérales (7) dont les extrémités libres (8) définissent une ouverture proximale (9),
- une membrane (3) qui est située au niveau des extrémités libres (8) des parois latérales (7) pour fermer de manière sensiblement hermétique l'ouverture proximale (9), délimitant ainsi une chambre (12) ;
- un conduit (4) connecté au boîtier rigide (2) et qui relie la chambre (12) à l'extérieur du dispositif d'injection (1),
la membrane (3) étant, lors d'une injection, destinée à être transpercée par une aiguille pour envoyer une dose de ladite substance dans le corps du patient au travers de la chambre (12) et du conduit (4),
les parois latérales (7) et le fond (6) du boîtier rigide (2) étant non transperçables par l'aiguille pour empêcher la pénétration de l'aiguille dans le corps du patient lors de son introduction dans la chambre (12),
le dispositif (1) comprenant en outre une enveloppe (15) en matériau élastomère qui recouvre le boîtier (2) **caractérisé en ce que** la membrane (3) fait partie intégrante de l'enveloppe (2), et le grade de l'élastomère utilisé pour la membrane (3) est différent de celui de l'enveloppe (15).

2. Dispositif selon la revendication 1 **caractérisé en ce que** le grade de l'élastomère de la membrane (3) est inférieur à celui de l'enveloppe (15).

3. Dispositif d'injection selon la revendication 2 **caractérisé en ce que** la membrane (3) est conformée pour constituer une surépaisseur s'étendant au-dessus des extrémités libres (8) des parois latérales (7), en direction opposée au fond (6) du boîtier (2) et reposant sur lesdites extrémités libres (8) pour être transperçable latéralement.

4. Dispositif d'injection selon la revendication 3 **caractérisé en ce que** la membrane (3) s'étend entièrement au dessus du boîtier (2), à partir des extrémités libres (8) des parois latérales (7).

5. Dispositif selon la revendication 4 **caractérisé en ce que** la membrane (3) prend la forme d'un cylindre avec des parois latérales (22) qui s'étendent dans le prolongement des parois latérales externes de l'enveloppe (15).

6. Dispositif selon l'une des revendications 3 à 5 **caractérisé en ce que** l'épaisseur de la membrane (3) est supérieure à l'épaisseur du reste de la membrane à l'aplomb de l'ouverture (9) du boîtier (2) pour définir un corps central (23) formant un épaulement.

7. Dispositif d'injection selon l'une quelconque des revendications précédentes **caractérisé en ce que** l'enveloppe (15) comporte une embase (25) qui est de forme élargie par rapport au fond (6) et qui est de forme convergente à partir de l'embase (25).

8. Dispositif d'injection selon la revendication 7 **caractérisé en ce que** le pourtour de l'embase (25) est de section transversale arrondie.

9. Dispositif d'injection selon l'une quelconque des revendications 1 à 10 **caractérisé en ce que** le boîtier (2) est réalisé en titane, et l'enveloppe (15) et la membrane (3) sont réalisées en matériau élastomère du type silicone par moulage autour du boîtier (2).

10. Dispositif d'injection selon l'une quelconque des revendications 1 à 11 **caractérisé en ce que** les extrémités libres (8) des parois latérales (7) comportent un biseau intérieur.

## Patentansprüche

1. Vorrichtung zur Arzneimittelinjektion, die dazu bestimmt ist, in eine subkutane Zone des Körpers eines Patienten implantiert zu werden, um Injektionen der Arzneimittel in den Körper durchzuführen, wobei die Vorrichtung (1) umfasst:
- ein starres hohles Gehäuse (2), das einen Boden (6) umfasst, von dem aus sich Seitenwände (7) erstrecken, deren freien Enden (8) eine proximale Öffnung (9) definieren,
- eine Membran (3), die sich im Bereich der freien Enden (8) der Seitenwände (7) befindet, um die proximale Öffnung (9) im Wesentlichen dicht zu verschließen und auf diese Weise eine Kammer (12) zu begrenzen;
- eine Leitung (4), die an das starre Gehäuse (2) angeschlossen ist und die Kammer (12) mit der Umgebung der Injektionsvorrichtung (1) verbindet,
wobei die Membran (3) bei einer Injektion dazu bestimmt ist, mit einer Nadel durchstoßen zu werden, um eine Dosis des Arzneimittels durch die Kammer (12) und die Leitung (4) in den Körper des Patienten zu schicken,
wobei die Seitenwände (7) und der Boden (6) des starren Gehäuses (2) nicht von der Nadel durchstoßen werden können, um so das Eindringen der Nadel in den Körper des Patienten bei ihrer Einführung in die Kammer (12) zu verhindern,
wobei die Vorrichtung (1) femer eine Hülle (15) aus einem elastomeren Material umfasst, die das Gehäuse (2) bedeckt, **dadurch gekennzeichnet, dass** die Membran (3) Bestandteil der Hülle (15) ist und dass sich der für die Membran (3) verwendete Elastomergrad von jenem der Hülle (15) unterscheidet.

2. Vorrichtung nach Anspruch 1, **dadurch gekennzeichnet, dass** der Elastomergrad der Membran (3) geringer als jener der Hülle (15) ist.

3. Injektionsvorrichtung nach Anspruch 2, **dadurch gekennzeichnet, dass** die Membran (3) derart geformt ist, dass sie eine Überdicke bildet, die sich über den freien Enden (8) der Seitenwände (7) in eine zum Boden (6) des Gehäuses (2) entgegengesetzte Richtung erstreckt und auf den freien Enden (8) aufliegt, um seitlich durchstoßbar zu sein.

4. Injektionsvorrichtung nach Anspruch 3, **dadurch gekennzeichnet, dass** sich die Membran (3) ausgehend von den freien Enden (8) der Seitenwände (7) vollständig über dem Gehäuse (2) erstreckt.

5. Vorrichtung nach Anspruch 4, **dadurch gekennzeichnet, dass** die Membran (3) die Form eines Zylinders mit Seitenwänden (22) annimmt, die sich in der Verlängerung der äußeren Seitenwände der Hülle (15) erstrecken.

6. Vorrichtung nach einem der Ansprüche 3 bis 5, **dadurch gekennzeichnet, dass** die Dicke der Membran (3), die sich senkrecht auf der Öffnung (9) des Gehäuses (2) befindet, größer als die Dicke der übrigen Membran ist, um auf diese Weise einen einen Absatz bildenden Mittelkörper (23) zu definieren.

7. Injektionsvorrichtung nach einem der vorhergehenden Ansprüche, **dadurch gekennzeichnet, dass** die Hülle (15) einen Sockel (25) umfasst, der im Vergleich zum Boden (6) eine erweiterte Form aufweist und eine, ausgehend vom Sockel (25), konvergierende Form hat.

8. Injektionsvorrichtung nach Anspruch 7, **dadurch gekennzeichnet, dass** der Umfang des Sockels (25) einen abgerundeten Querschnitt hat.

9. Injektionsvorrichtung nach einem der Ansprüche 1 bis 8, **dadurch gekennzeichnet, dass** das Gehäuse (2) aus Titan hergestellt ist, und dass die Hülle (15) und die Membran (3) aus einem elastomeren Material vom Typ Silikon durch Formguss um das Gehäuse (2) hergestellt sind.

10. Injektionsvorrichtung nach einem der Ansprüche 1 bis 9, **dadurch gekennzeichnet, dass** die freien Enden (8) der Seitenwände (7) eine innere Abschrägung aufweisen.

## Claims

1. Device for injecting medical substances and designed to be implanted in a subcutaneous zone of the body of a patient in order to inject said substances into the body, the device (1) comprising:
- a hollow rigid housing (2) with a base (6) from which there extend side walls (7) whose free ends (8) define a proximal opening (9),
- a membrane (3) which is situated at the free ends (8) of the side walls (7) in order to close the proximal opening (9) in a substantially hermetic manner, thereby delimiting a chamber (12),
- a conduit (4) connected to the rigid housing (2) and connecting the chamber (12) to the outside of the injection device (1),
the membrane (3) being designed to be pierced by a needle, at the time of an injection, in order to deliver a dose of said substance into the body of the patient via the chamber (12) and the conduit (4),
the side walls (7) and the base (6) of the rigid housing (2) being non-pierceable by the needle, so as to prevent the needle penetrating into the body of the patient during its introduction into the chamber (12),
the device (1) additionally comprising an envelope (15) of elastomer material which covers the housing (2), **characterized in that** the membrane (3) forms an integral part of the envelope (2) and the grade of the elastomer used for the membrane (3) is different from that for the envelope (15).

2. Device according to Claim 1, **characterized in that** the grade of the elastomer for the membrane (3) is less than that for the envelope (15).

3. Injection device according to Claim 2, **characterized in that** the membrane (3) is shaped to constitute an additional thickness extending over the free ends (8) of the side walls (7) in the direction away from the base (6) of the housing (2) and resting on said free ends (8) in such a way as to be pierceable from the sides.

4. Injection device according to Claim 3, **characterized in that** the membrane (3) extends completely above the housing (2) from the free ends (8) of the side walls (7).

5. Device according to Claim 4, **characterized in that** the membrane (3) takes the form of a cylinder with side walls (22) which extend in the continuation of the outer side walls of the envelope (15).

6. Device according to one of Claims 3 to 5, **characterized in that** the thickness of the membrane (3) in line with the opening (9) of the housing (2) is greater than the thickness of the rest of the membrane in order to define a central body (23) forming a shoulder.

7. Injection device according to any one of the preceding claims, **characterized in that** the envelope (15) includes a seat (25) which is wider than the base (6) and which is of a shape converging from the seat (25).

8. Injection device according to Claim 7, **characterized in that** the circumference of the seat (25) is of rounded cross section.

9. Injection device according to any one of Claims 1 to 10, **characterized in that** the housing (2) is made of titanium, and the envelope (15) and the membrane (3) are made of an elastomer material of the silicone type by being moulded around the housing (2).

10. Injection device according to any one of Claims 1 to 11, **characterized in that** the free ends (8) of the side walls (7) have an inner bevel.
